# EUROPÄISCHE PATENTSCHRIFT

(11) **EP 3 727 216 B1**
(45) Veröffentlichungstag und Bekanntmachung des Hinweises auf die Patenterteilung: **05.01.2022**
(21) Anmeldenummer: 18829402.9
(22) Anmeldetag: 21.12.2018
(51) Int. Cl.: A61F 5/01

(54) **HARTRAHMEN MIT VERSCHWENKBARER BRÜCKE**
HARD FRAME WITH PIVOTING BRIDGE
CADRE DUR COMPRENANT UN PONT PIVOTABLE

(30) Priorität: 22.12.2017 DE 102017223757
(43) Veröffentlichungstag der Anmeldung: 28.10.2020
(73) Patentinhaber: Bauerfeind AG, 07937 Zeulenroda-Triebes (DE)
(72) Erfinder: GÖRNERT, Florian, 07937 Zeulenroda-Triebes (DE); HEBENSTREIT, Sandro, 07937 Zeulenroda-Triebes (DE); BAUERFEIND, Hans B., 07937 Zeulenroda-Triebes (DE)
(74) Vertreter: Wohlfahrt, Jan Günther
(86) Internationale Anmeldenummer: PCT/EP2018/086667
(87) Internationale Veröffentlichungsnummer: WO 2019/122364

(56) Entgegenhaltungen:
- EP-A1- 3 216 429
- WO-A1-2013/040375
- US-A- 5 092 320
- US-A- 5 286 250
- US-A1- 2013 041 300

## Beschreibung

Die vorliegende Erfindung betrifft medizinische Gelenkorthesen zur Führung oder Stützung der Gelenkfunktion an Extremitätengelenken, besonders Kniegelenkorthesen. Die Erfindung stellt einen verbesserten starren Stützrahmen (Hartrahmen) zur Ausbildung einer solchen Gelenkorthese zur Verfügung, wobei der Hartrahmen besonders eine Fassung mit kipp- oder verschwenkbarer Rahmenbrücke aufweist.

Erfindungsgemäße Gelenkorthesen mit starrem Hartrahmen sind beispielsweise aus der EP 3 216 429 A1 bekannt. Ein Gelenkorthesenhartrahmen zur Überbrückung und damit Stützung und Führung eines Extremitätengelenks, beispielsweise des Kniegelenks, wird gattungsgemäß durch zwei sich gegenüberliegende Gelenkschienen gebildet, welche im angelegten Zustand der Orthese das zu stützende oder zu führende Extremitätengelenk überbrücken. Diese Gelenkschienen weisen jeweils ein Gelenk auf, das uniaxial oder multiaxial ausgebildet ist, um die anatomische Gelenkbewegung des Extremitätengelenks weitgehend nachzubilden und dieser zu folgen. Diesseits und jenseits des Gelenks sind die Arme der Gelenkschienen mit einer körpernahen (proximalen) beziehungsweise körperfernen (distalen) Fassung in Verbindung. Diese Fassungen umgreifen jeweils den körpernahen beziehungsweise körperfernen Extremitätenabschnitt fest, um eine mechanische Kopplung mit der Extremität, Arm oder Bein, Hand oder Fuß, zu erreichen, um die gewünschte Stützfunktion der Gelenkschienen auf das überbrückte Gelenk der Extremität zu vermitteln. Extremitätenfassungen und Gelenkschienen bilden im Wesentlichen die Einheit dieses gattungsgemäßen Hartrahmens. Solche Hartrahmen werden gattungsgemäß entweder direkt über entsprechende Vergurtungen, insbesondere im Bereich der Extremitätenfassungen mit der Extremität verbunden oder alternativ oder zusätzlich auf an der Extremität eng anliegenden Gelenkbandagen aufgerüstet, das heißt mit diesen mechanisch verkoppelt.

Problematisch bei solchen bekannten Hartrahmen ist, dass es im Bereich der Fassung des jeweiligen Extremitätenabschnitts, das heißt bei Kniegelenkorthesen besonders an dem Unterschenkel, bei Verwendung zu einer mechanischen Überbeanspruchung, insbesondere durch Druck- oder Scherbelastung, der darunterliegenden Gewebeabschnitte kommen kann. Dies führt im günstigen Fall zu unangenehmem Druckgefühl oder Schmerzen beim Träger der Orthese, im ungünstigen Fall zu signifikanten Gewebsverletzungen, die dazu führen, dass die Orthese nicht mehr getragen werden kann.

Andererseits hat sich gezeigt, dass in vielen Fällen zur Ausbildung einer gut stützenden Orthese die beiden seitlichen gelenkübergreifenden Gelenkschienen in dem Hartrahmen durch starre Brücken miteinander verbunden werden müssen, wobei diese Rahmenbrücken gleichzeitig Teil der Extremitätenfassung sind. Gerade solche mechanisch notwendigen Rahmenbrücken können beim Tragen der Orthese in benachbarte Gewebeabschnitte der Extremität drücken. Kompensierende Lösungen bestehen darin, an den Rahmenbrücken Polster vorzusehen, welche den Druck auf die darunterliegenden Gewebe verteilen und vermindern. Solche Lösungen sind ungünstig, weil sie gleichzeitig die gute ortsfeste Fixierung des Hartrahmens an der Extremität verhindern, eine unerwünschte Migration der Orthese verursachen, die notwendige mechanische Krafteinleitung oder -koppelung von Hartrahmen und Extremität vermindern und damit die Stützwirkung der Orthese reduzieren oder gänzlich unwirksam machen. Ähnliche Funktionszusammenhänge und damit verbundene Probleme existieren bei Kniegelenkorthesen und auch bei Handgelenkorthesen und Sprunggelenkorthesen sowie auch bei Ellbogengelenkorthesen.

Es bestand daher das technische Problem, einen an sich bekannten Hartrahmen als Bestandteil für solche Gelenkorthesen so weiterzubilden, dass diese problemlos getragen werden können und die volle Funktionalität der Orthesen erhalten werden kann, ohne dass es zu unerwünschter oder schädlicher Druck- oder Scherbelastung an den benachbarten Geweben der Extremität kommt und wobei gleichzeitig eine unerwünschte Migration der Orthese wirksam unterdrückt wird.

Das zugrundeliegende technische Problem wird gelöst durch einen Hartrahmen für eine Extremitätengelenkorthese gemäß Anspruch 1. Dieser weist besonders zwei sich gegenüberliegende längs verlaufende Gelenkschienen auf, die jeweils von ihrem zentralen, insbesondere multiaxialen, Gelenk ausgehend, zwei sich im angelegten Zustand in proximaler Richtung und distaler Richtung erstreckende Gelenkarme besitzen. Die Gelenkarme der Gelenkschienen sind über Brücken miteinander verbunden, um den Hartrahmen für die Orthese zu bilden. Die Brücken sind bogenförmig ausgeformt, um den jeweiligen proximalen oder distalen Extremitätenabschnitt eng zu umfassen. Sie bilden einen wesentlichen Teil der Extremitätenfassung. Zur Erzielung des vollständigen Kraftschlusses an der Fassung mit dem jeweiligen Extremitätenabschnitt ist bevorzugt ein Spanngurt vorgesehen; alternativ oder zusätzlich bevorzugt ist der Hartrahmen auf eine eng anliegende elastische Gelenkbandage ausgerüstet, um an der Extremität fixiert zu werden. Indikationen für solche Hartrahmenorthesen sind neben Gelenkarthrose oder Gelenkfehlstellungen vor allem auch die Funktionelle Unterstützung nach Operationen zur Behandlung von Bänderrissen oder Knorpeldefekten.

Erfindungsgemäß ist nun vorgesehen, dass zumindest einer der die Gelenkschienen verbindenden Brückenbögen an den Armen der Gelenkschienen, die er jeweils verbindet, schwenkbar gelagert ist. Erfindungsgemäß ist dabei vorgesehen, dass diese Lagerung die Verschwenkung dieser Rahmenbrücke zumindest in derjenigen Verschwenkachse erlaubt, welche im Wesentlichen parallel zu der Verbindungslinie zwischen den beiden über diese Brücke verbundenen Gelenkarmen liegt, das heißt dass sich diese Verschwenkachse in Richtung der Verbindung der beiden jeweils über diesen Brückenbogen verbundenen Gelenkarme erstreckt. Die erfindungsgemäße Lagerung mindestens einer der beiden die Arme der Gelenkschienen verbindenden Brücken erlaubt eine günstige Verkippung der bogenförmigen Brücke in Richtung der Verschwenkung der Gelenkschienen, das heißt im angelegten Zustand in Richtung der Beugung oder Streckung des Gelenks. Dabei erlaubt die Verkippung der Brücke besonders, dass bei Beugung oder Streckung auftretende zwangsläufige Änderungen der Relativposition der Fassung zu der Extremität, aufgrund der Elastizität des Gewebes bei Beugung oder Streckung einerseits und aufgrund der Unelastizität des stützenden Hartrahmens andererseits kompensiert und ausgeglichen werden können. Eine Reibbewegung der Hartrahmenbrücke an der Extremitätenfassung durch auftretende Scherkräfte und eine Druckbelastung durch die Kanten des Brückenbogens aufgrund ungünstigerer Anlenkwinkel des Brückenbogens zu der Oberfläche der Extremität vermindert oder gänzlich vermieden werden können. Dies ist besonders relevant und effizient bei Verwendung eines insgesamt starren, das heißt unelastischen und verwindungssteifen, Brückenbogens.

Demgemäß ist Gegenstand der Erfindung ein Hartrahmen für eine gelenküberbrückende Extremitätengelenkorthese, enthaltend zwei sich gegenüber liegende längsverlaufende Gelenkschienen, die über deren proximale Gelenkarme über einen querenden proximalen Brückenbogen miteinander verbunden sind und dort eine proximale Extremitätenfassung bilden und weiter über deren distale Gelenkarme über einen querenden distalen Brückenbogen miteinander verbunden sind und dort eine distale Extremitätenfassung bilden, dadurch gekennzeichnet, dass mindestens einer der Brückenbögen insbesondere starr ist und erfindungsgemäß jeweils an Gelenkarmenden der verbundenen Gelenkarme in Gelenken in mindestens einer Verschwenkachse schwenkbar gelagert ist, wobei sich diese Verschwenkachse in Richtung der Verbindung der beiden über diesen Brückenbogen verbundenen Gelenkarme erstreckt gemäß Anspruch 1.

Besonders ist vorgesehen, dass das Gelenk zwischen den jeweiligen Enden des gelenkig gelagerten Brückenbogens und der damit verbundenen Lenkarmenden im einfachsten Fall durch einen Gelenkbolzen realisiert ist, welcher so ein einfaches Axialgelenk bildet. Im einfachsten Fall ist dazu das Flachmaterial des Gelenkarmendes mit dem Flachmaterial des Endes des Brückenbogens in einem Punkt vernietet oder verschraubt oder in ähnlicher Weise verbunden, um dieses Axialgelenk zu bilden. Bevorzugt ist dabei vorgesehen, dass sich Gelenkarmende und das damit verbundene Ende des Brückenbogens flach überlappen und bei der vorgesehenen Verschwenkung des Brückenbogens gegen das Gelenkarmende dort aneinander gleiten. Dadurch wird insbesondere auch die Richtung der Verschwenkachse festgelegt, und zwar senkrecht zu der Fläche des flachen Gelenkarmendes, worauf das Ende des Brückenbogens gleitet.

Demgemäß ist Gegenstand der Erfindung bevorzugt auch ein erfindungsgemäßer Hartrahmen, wobei die Gelenke für die Verschwenkung in der mindestens einen Verschwenkachse jeweils zwischen Endabschnitten des starren Brückenbogens und den damit überlappenden Gelenkarmenden gebildet sind, wobei die Endabschnitte mit den Gelenkarmenden jeweils mit einem Achsbolzen drehbar miteinander verkoppelt sind.

In einer ersten Variante verläuft der proximale Brückenbogen im angelegten Zustand auf der Vorderseite (Streckseite) der Extremität; in der Ausführung als oder in einer Kniegelenksorthese also im Bereich des Schienbeins. In einer alternativen Variante dazu verläuft der proximale Brückenbogen im angelegten Zustand auf der Rückseite (Beugeseite) der Extremität; in der Ausführung als oder in einer Kniegelenksorthese also im Bereich der äußeren und inneren Wadenmuskeln und liegen dort besonders auf den Muskelbäuchen, die als Weichteilpolster bei der Krafteinkopplung dienen, fest auf.

In einer Variante verläuft der distale Brückenbogen im angelegten Zustand auf der Vorderseite (Streckseite) der Extremität; in der Ausführung als oder in einer Kniegelenksorthese also im Bereich des Geraden Oberschenkelmuskels sowie des Äußeren und des Inneren Oberschenkelmuskels und liegen dort besonders auf den Muskelbäuchen, die als Weichteilpolster bei der Krafteinkopplung dienen, fest auf. In einer alternativen Variante dazu verläuft der distale Brückenbogen im angelegten Zustand auf der Rückseite (Beugeseite) der Extremität; in der Ausführung als oder in einer Kniegelenksorthese also im Bereich des Halssehnigen Muskels, des Zweiköpfigen Oberschenkelmuskels und des Großen Anziehermuskels und liegen dort besonders auf den Muskelbäuchen, die als Weichteilpolster bei der Krafteinkopplung dienen, fest auf. Besonders bevorzugt verläuft der distale Brückenbogen des Hartrahmens an der Vorderseite, und der proximale Brückenbogen verläuft an der Rückseite der Extremität, in der Ausführung als oder in einer Kniegelenksorthese also im Bereich der Wadenmuskeln.

Die Verschwenkung erfolgt bevorzugt in derjenigen Rotationsrichtung, dass die Oberkante des Brückenbogens weg von dem überbrückten Gelenk kippen kann, besonders um eine bei Beugung der Extremität auftretende Verkürzung der wirksamen Länge des Hartrahmens an der Rückseite (Beugeseite) der Extremität und/oder eine auftretende Verlängerung der wirksamen Länge des Hartrahmens an der Vorderseite (Streckseite) der Extremität zu kompensieren. So können erfindungsgemäß ansonsten dadurch auftretende Druckbelastungen durch die Kanten des insbesondere starren Brückenbogens vermieden werden. Bei einer Ausführung des Hartrahmens als oder in einer Kniegelenksorthese ist insbesondere die proximale Brücke, welche im angelegten Zustand den Unterschenkel, insbesondere im Bereich der Rückseite, das heißt an der Wade, umfasst, schwenkbar. Die Verschwenkbarkeit erlaubt dabei, dass die Brücke aus einer Normallage heraus nach proximal ausweichen kann, um eine Scher- und Druckbelastung an der Wade bei Beugung des Knies zu verhindern oder zu reduzieren.

Bevorzugt ist nur die distale Brücke verschwenkbar, und bevorzugt ist dabei die proximale Brücke starr mit den beiden proximalen Gelenkarmen der Gelenkschienen verbunden und weiter bevorzugt mit diesen als eine einstückige Einheit ausgebildet. Alternativ ist ausschließlich, oder in einer Variante zusätzlich, die proximale Brücke verschwenkbar, und die distale Brücke kann dabei starr mit den beiden distalen Gelenkarmen der Gelenkschienen verbunden und weiter bevorzugt mit diesen als eine einstückige Einheit ausgebildet sein. In einer weiteren Ausführung sind die proximale Brücke und die distale Brücke an den jeweiligen Gelenkarmen der Gelenkschienen schwenkbar gelagert.

Besonders bevorzugt ist aber eine Ausführung, in der zumindest immer die insbesondere starre distale Brücke verschwenkbar ist, die besonders im Zusammenhang mit einer Knieorthese im angelegten Zustand an den Wadenmuskeln liegt. In einer alternativen Ausgestaltung als oder in einer Handgelenkorthese ist besonders vorgesehen, dass zumindest die insbesondere starre proximale Brücke verschwenkbar ist, die im angelegten Zustand an den Unterarmmuskeln liegt. In einer weiteren Ausgestaltung als oder in einer Handgelenkorthese ist alternativ besonders vorgesehen, dass zumindest die insbesondere starre distale Brücke verschwenkbar ist, die im angelegten Zustand an der Mittelhand anliegt.

Erfindungsgemäß ist vorgesehen, dass der Grad der Verschwenkung des Brückenbogens an den jeweils verbundenen Gelenkarmenden in der mindestens einen Verschwenkachse limitiert ist. Die Verschwenkungslimitierung erlaubt nur eine gewisse Bewegung des Brückenbogens an der Extremitätenfassung, um besonders zwar die vorgenannten Relativbewegungen der Extremität an der Fassung zu der Orthese bei Beugung oder Streckung zu kompensieren, aber dennoch eine ausreichend starke mechanische Kopplung zwischen Extremität und Hartrahmen zu sichern. Die Verschwenkungslimitierung ist in einer ersten Variante durch einen harten Anschlag realisiert. Erfindungsgemäß ist an den jeweiligen Enden des Brückenbogens ein Langloch vorgesehen, worin ein Gleitbolzen, welcher mit dem jeweiligen Gelenkarmende ortsfest verbunden ist, gleiten kann. Alternativ oder zusätzlich ist das Langloch an dem Gelenkarmende ausgebildet, und der Gleitbolzen ist an dem Ende des Brückenbogens ausgebildet. Alternativ oder zusätzlich ist ein mechanischer Anschlag an dem Seitarmende und/oder an dem Brückenbogen vorgesehen, wobei eine Außenkante des einen Gelenkelements mit einem Vorsprung des anderen Gelenkelements in Eingriff kommt, um die Verschwenkungslimitierung zu bilden.

Bevorzugt ist eine Limitierung der Verschwenkung der Brücke aus der Neutrallage heraus auf etwa 20° oder auf etwa 15°, vorgesehen. Besonders bevorzugt ist aber vorgesehen, den Winkel der Verschwenkungslimitierung je nach Einsatz und Anwendungsfall des Hartrahmens, das heißt besonders je nach Bewegungsprofil des Trägers und/oder nach Therapieziel unterschiedlich zu gestalten. Das heißt, besonders kann die Verschwenkungslimitierung weniger restriktiv, besonders auf 12° bis 20° begrenzt sein, wenn eine große Beweglichkeit beim Tragen der Orthese erhalten werden soll oder das verfolgte Therapieziel nicht besonders streng ist. Andererseits kann die Verschwenkungslimitierung restriktiv und auf wenige Winkelgrade, besonders 5° bis 12° beschränkt sein, wenn eine gesteigerte Stützwirkung durch die Orthese gewünscht ist oder die Beweglichkeit des Trägers ohnehin eingeschränkt ist. Eine variable Verschwenkungslimitierung, die bis zur optionalen völligen Blockierung des Gelenks reichen kann, ist in an sich bekannter Weise erreichbar. Bevorzugt sind bei einer Ausführung mit einem in einem Langloch gleitenden Bolzen, dass bei an sich konstanter Langlochbogenlänge verschiedene Einsteckpunkte eines oder mehrerer Gleitbolzen oder alternativ oder zusätzlich die Ausbildung verschieden langer Langlöcher, welche wahlweise durch Einstecken des Gleitbolzens ausgewählt werden können.

In einer bevorzugten Ausgestaltung ist alternativ oder zusätzlich vorgesehen, dass die Gelenkbewegung an dem zwischen Gelenkarmende und Brückenbogen gebildeten Gelenk mechanisch gehemmt ist, um der Verschwenkung des Brückenbogens einen mechanischen Widerstand entgegenzusetzen. Damit wird bevorzugt erreicht, dass auf der einen Seite die Druckbelastung des Gewebes an der Extremitätenfassung ausreichend reduziert wird, auf der anderen Seite aber die Brücke an der Fassung noch ausreichend mechanischen Widerstand für eine funktionsgerechte mechanische Kopplung des Hartrahmens mit der Extremität gewährleistet bleibt. Diese Schwenkhemmung wird bevorzugt durch mechanische Reibung zwischen den Endabschnitten des Brückenbogens und der jeweils damit über das Gelenk verbundenen Gelenkarmenden erreicht. Bevorzugt ist dazu vorgesehen, dass zumindest eines der beiden Gelenkelemente einen Reibbelag aufweist, wodurch die mechanische Reibung am Gelenk modifiziert, bevorzugt verstärkt wird. Alternativ oder zusätzlich ist dazu vorgesehen, dass Gelenkschiene und überlappender Endabschnitt der Brücke um den Achsbolzen herum gerändelt oder mit einer Verzahnung versehen sind, um die Reibung durch diese Oberflächenstrukturierung verstärken. Dabei ist besonders vorgesehen, dass die Verzahnung eine Rastung bildet, so dass sich die Brücke erst ab einem bestimmten Krafteintrag eine Raste weiterverschwenkt und somit die übliche starre Führungsfunktion der Brücke im Rahmen nur leichter Krafteinträge, welche den Rastwiderstand noch nicht überwinden, konstant erhalten bleibt.

In einer alternativen oder zusätzlichen bevorzugten Ausgestaltung ist vorgesehen, dass das Gelenk zwischen Gelenkarmende und Brückenbogen ausschließlich oder zusätzlich durch einen zwischen diesen beiden Elementen ausgebildeten Elastomerblock gebildet wird. Dieser dient vorteilhafterweise zum einen einer mechanischen Hemmung der Schwenkbewegung, zum anderen aber auch einer sanft einsetzenden, vom Verschwenkungswinkel abhängigen Verschwenkungslimitierung. Dabei ist der Elastomerblock ein Torsionsgelenk mit Feder- und Dämpfungswirkung. Bevorzugte Elastomermaterialien sind Silikonelastomere und Polyurethanelastomere. In einer besonderen Variante sind thermosensible Polymere vorgesehen, die mit zunehmender mechanischer Beanspruchung nachgiebiger werden. Dadurch wird eine automatische Anpassung von Verschwenkgrad und Verschwenkungshemmung an den jeweiligen Bewegungszustand ermöglicht. In analogen alternativen Ausgestaltungen davon können einfache Axialgelenke mit angekoppelter Spiralfeder vorgesehen sein.

Erfindungsgemäß ist vorgesehen, dass an dem Hartrahmen mindestens ein Gelenkarmende, besonders wo das Gelenkarmende erfindungsgemäß mit dem verschwenkbaren Brückenbogen verbunden ist, als Blattfeder, das heißt elastisches Federblatt ausgebildet ist. Dies erlaubt vorteilhafterweise eine elastische Verkippung des Gelenkarmendes aus der Ebene der Längserstreckung der Gelenkschiene, das heißt besonders aus der primären Schwenkebene des Gelenks der Gelenkschiene heraus. Dies erlaubt insbesondere im Zusammenhang mit einem weiteren fixierbaren Kippgelenk in der Gelenkschiene, mit dem mindestens einer der Gelenkarme der Gelenkschiene aus der primären Verschwenkebene des Gelenks der Gelenkschiene verkippt werden kann, Fehlstellungen am Extremitätengelenk quer zu der primären Beuge- und Streckrichtung des Gelenks zu kompensieren oder aktiv das Extremitätengelenk in dieser Verkippung zu führen. Ein solches fixierbares Kippgelenk kann als zusätzliches Axialgelenk in einem oder mehreren der Gelenkarme ausgebildet sein, wobei die Achse dieses Kippgelenks in der Ebene der Gelenkschiene und quer zur Längserstreckung der Gelenkschiene orientiert ist. Alternativ oder zusätzlich kann das fixierbares Kippgelenk neben, in oder als Teil des primären Schwenkgelenks der Gelenkschiene ausgebildet sein.

Dabei ist besonders vorgesehen, dass das fixierbare Kippgelenk die fest einstellbare Verkippung des Gelenkarms aus der Verschwenkebene der Gelenkschiene erzwingt, das bevorzugt zusätzlich vorgesehene Federblatt an dem Gelenkarmende des jeweils verkippten Gelenkarms diese erzwungene Verkippung kompensiert, um den Brückenbogen auch trotz dieser seitlichen Verkippung streng quer zur Längserstreckung der Extremität auszurichten. Dadurch wird ermöglicht, dass die durch diesen Brückenbogen gebildete Extremitätenfassung stets quer zu der Längserstreckung der Extremität und damit mit an dieser Stelle geringstmöglichen Umfang verläuft. Eine unerwünschte Migration des Hartrahmens und damit der Gelenkorthese durch Verrutschen der Fassung wird so entgegengewirkt.

Die hierin beschriebene Hartrahmenkonstruktion mit kippbarer Brücke zwischen den Gelenkschienen ist besonders gut anwendbar im Zusammenhang mit Kniegelenksorthesen, wobei bevorzugt zumindest der Brückenbogen für die distale Extremitätenfassung, das heißt für die Fassung des Unterschenkels, starr ausgebildet ist und dieser Brückenbogen erfindungsgemäß an den durch ihn verbundenen distalen Gelenkarmenden der beiden Gelenkschienen in der erfindungsgemäßen Weise verschwenkbar gelenkig angeordnet ist. Die besonders am Unterschenkel auftretenden Druckbelastungen und Scherkräfte bei Beugung oder Streckung des Knies werden durch den verschwenkbaren Brückenbogen weitgehend oder vollständig kompensiert, sodass eine sichere Unterschenkelfassung erreicht wird, welche gleichzeitig für den Träger mechanisch wenig belastend ist. Ein besonderer Gegenstand der vorliegenden Erfindung ist daher eine Kniegelenksorthese, welchen den erfindungsgemäßen Hartrahmen enthält.

In einer alternativen Ausgestaltung ist die Gelenkorthese, welche den erfindungsgemäßen Hartrahmen enthält, eine Handgelenkorthese. In einer weiteren alternativen Ausgestaltung ist die Gelenkorthese, welche den erfindungsgemäßen Hartrahmen enthält, eine Ellenbogengelenkorthese. In einer weiteren alternativen Ausgestaltung ist die Gelenkorthese, welche den erfindungsgemäßen Hartrahmen enthält, eine Sprunggelenkorthese oder eine Fußhebeorthese.

Die Erfindung wird durch die nachfolgenden Ausführungsbeispiele, welche in den Figuren illustriert sind, näher erläutert. Die Figuren zeigen dabei bevorzugte Ausgestaltungen, welche neben den erfindungsgemäßen Erfindungsmerkmalen weitere optionale Merkmale zeigten. Letztere sind nicht beschränkend für die vorliegende Erfindung zu verstehen, sondern zeigen nur praktische und bevorzugte Ausführungsvarianten.

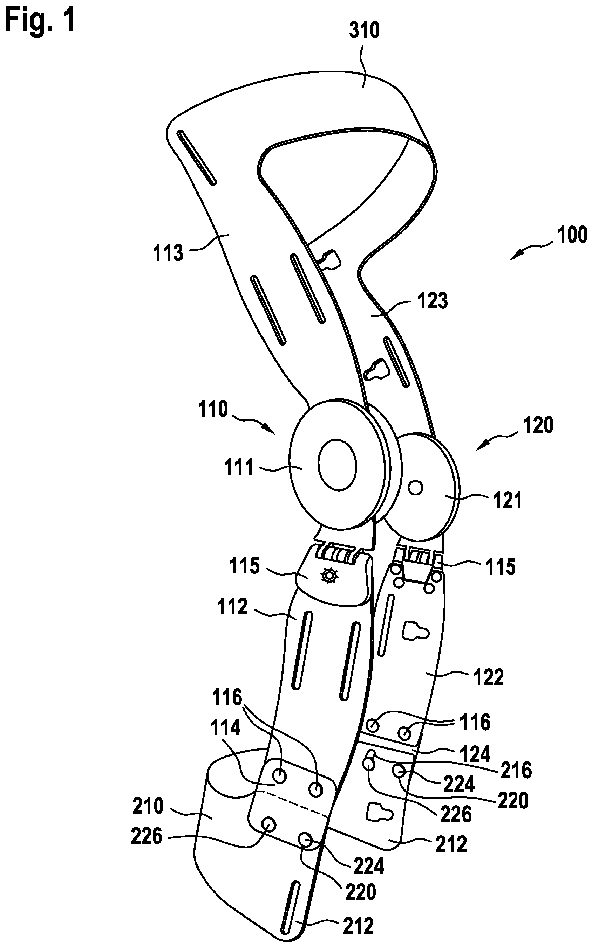

Figur 1 zeigt eine perspektivische Ansicht einer Ausführungsform des erfindungsgemäßen Hartrahmens 100 als oder für eine Kniegelenkorthese. Der Hartrahmen 100 setzt sich im Wesentlichen zusammen aus zwei sich gegenüberliegenden, das Gelenk übergreifenden Gelenkschienen 110, 120. Die außenliegende Gelenkschiene 120 weist im Wesentlichen denselben prinzipiellen Aufbau wie die innere Gelenkschiene 110 auf. Die Gelenkschienen 110, 120 besitzen jeweils zentrale Gelenke 111, 121, die insbesondere als multiaxiales Gelenk ausgebildet sind, um die Gelenkbewegung des zu stützenden Kniegelenks anatomisch nachzubilden. Bei der Gelenkschiene 110 gehen von dem zentralen Gelenk 111 der proximale Gelenkarm 113 und der distale Gelenkarm 112 aus. Diese sind über das Gelenk 111 gekoppelt und dort in einer primären Schwenkebene der Gelenkschiene 110 gegeneinander verschwenkbar, um der Gelenkbewegung zu folgen. Analog gehen bei der Gelenkschiene 120 von dem zentralen Gelenk 121 der proximale Gelenkarm 123 und der distale Gelenkarm 122 aus, die über das Gelenk 121 gekoppelt und dort in einer primären Schwenkebene der Gelenkschiene 120 gegeneinander verschwenkbar sind, um der Gelenkbewegung zu folgen. Die Gelenkschienen 110, 120 sind jeweils an den Enden der proximalen Gelenkarme 113, 123 über eine proximale Brücke 310 und an den Enden der distalen Gelenkarme 112, 122 über eine distale Brücke 210 miteinander verbunden, um schließlich den Hartrahmen 100 zu bilden.

In der dargestellten Ausführung ist zusätzlich jeweils an den distalen Gelenkarmen 112 und 122 ein fixierbares Kippgelenk 115 vorgesehen. Dieses fixierbare Kippgelenk 115 ermöglicht eine Verkippung des jeweiligen Gelenkarms 112, 122 aus der primären Schwenkebene seiner Gelenkschiene 110, 120 heraus.

Erfindungsgemäß ist in der dargestellten Ausführung die distale Brücke 210 jeweils an den Gelenkarmenden 114 und 124 der distalen Gelenkarme 112 verschwenkbar gelagert. Dabei ist erfindungsgemäß das Gelenkarmende 114 über einen ortsfesten Niet, der als Achsbolzen 224 dient, mit der einem überlappenden Endabschnitt 212 der Brücke 210 verbunden und dort in einer in dem Endabschnitt 212 ausgebildeten Lagerbuchse drehbar gelagert ist und dadurch ein Axialgelenk 220 gebildet, woran die Brücke 210 an dem Gelenkarmende 114 verschwenkbar gelagert ist. Ebenso ist das Gelenkarmende 124 über einen ortsfesten Niet, der als Achsbolzen 224 dient, mit der einem überlappenden Endabschnitt 212 der Brücke 210 verbunden und dadurch dort ebenfalls ein Axialgelenk 220 gebildet, worüber die Brücke 210 auch an dem Gelenkarmende 124 verschwenkbar gelagert ist.

Zur Realisierung einer bevorzugten Verschwenkungslimitierung an den Gelenken 220 ist jeweils ein weiterer Niet 226 ortsfest mit dem jeweiligen Gelenkarmende 114, 124 verbunden und gleitet als Gleitzapfen 226 jeweils in einem Langloch 216, welches in dem jeweiligen überlappenden Endabschnitt 212 der gelagerten Brücke 210 ausgebildet ist. Das Langloch 216 dient als Anschlag für den Gleitzapfen 226 und begrenzt so den Grad der Verschwenkung der Brücke 210.

Weiter ist in der dargestellten Ausführungsform das jeweilige Gelenkarmende 114 und 124 als Federblatt ausgebildet, welches hier mit dem Gelenkarm 112 und 122 jeweils über Nieten 116 mechanisch ortsfest verkoppelt ist. In dem Federblatt sind entsprechend auch der Achsbolzen 224 und der Gleitzapfen 226 ausgebildet.

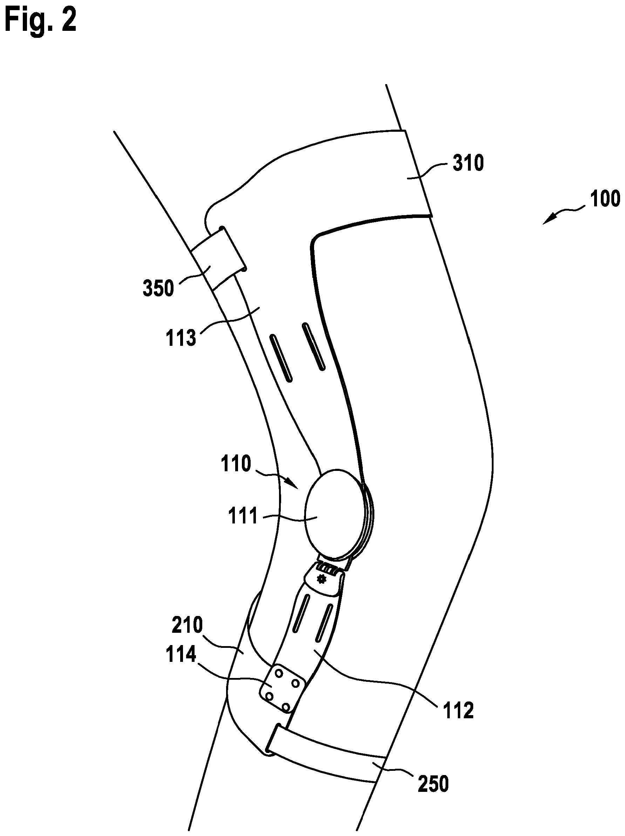

Figur 2 zeigt eine perspektivische Ansicht einer Ausführung als Kniegelenkorthese nach Figur 1, angelegt über dem schematisch angedeuteten Knie. Die Bezugszeichen gelten entsprechend. Die proximale Brücke 310 bildet zusammen mit dem Spanngurt 350 eine im angelegten Zustand den Oberschenkel umgreifende proximale Extremitätenfassung 300. Die distale Brücke 210 bildet zusammen mit dem Spanngurt 250 eine im angelegten Zustand den Unterschenkel umgreifende distale Extremitätenfassung 200. In der dargestellten Ausführung ist der Hartrahmen 100 so über die Gurte 250, 350 und die Brücken 210, 310 mechanisch fest mit der Extremität verbunden, um ausreichende Krafteinleitung für die Stützfunktion der Orthese zu erreichen.

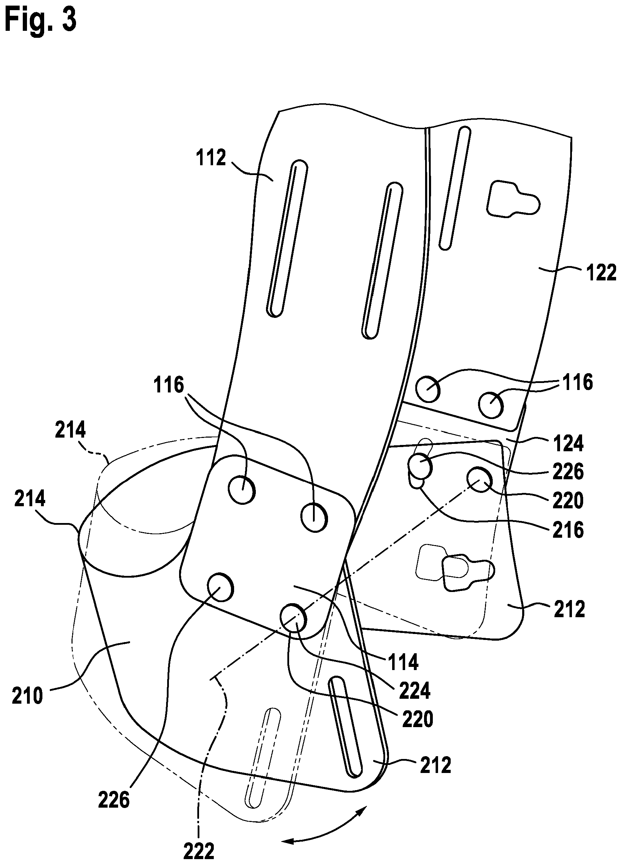

Die Figur 3 zeigt eine Detailansicht der Ausführung nach Figur 1 und 2. Der distale Brückenbogen 210 kann bei Benutzung erfindungsgemäß in den Gelenken 220 jeweils um die Verschwenkachse 222 rotieren. Die jeweiligen Verschwenkachsen 222 der Gelenke 220 sind schematisch dargestellt. Die Verschwenkachsen 222 verlaufen jeweils im Wesentlichen lotrecht zur Ebene der flachen Gelenkarmenden 114, 124. Insbesondere die Oberkante 214 des starren Brückenbogens 210 kann so bei Benutzung temporär nach distal abkippen (strichlierte Form), um spezifisch eine Scher- und Druckbelastung an dem Unterschenkel einer angelegten Knieorthese, die bei Bewegung, vor allem bei der Beugung des Knies auftritt, kompensieren.

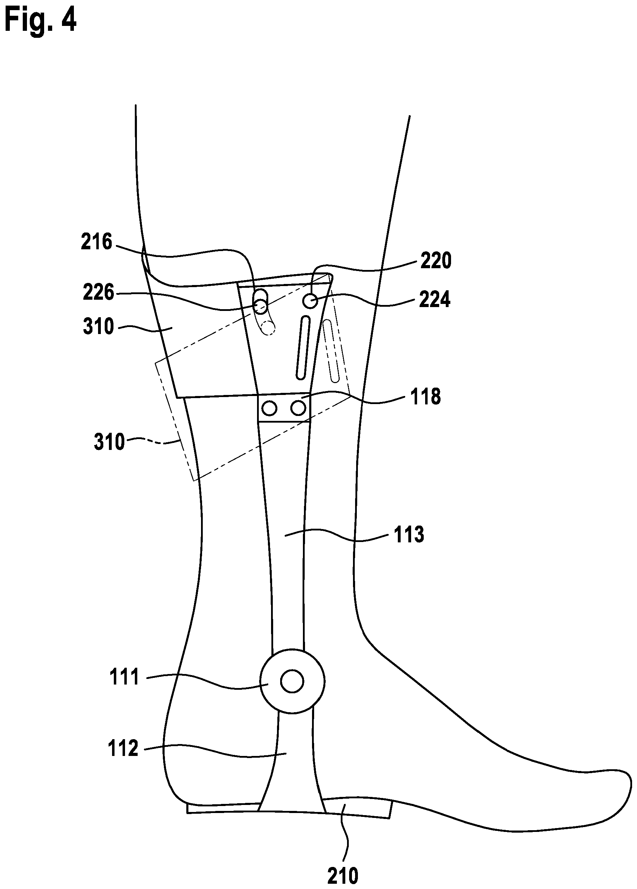

Figur 4 zeigt eine Ausführung als Sprunggelenkorthese. Die Bezugszeichen aus den Figuren 1 bis 3 gelten für entsprechende Strukturen. In dem Hartrahmen nach Figur 4 ist der proximale Brückenbogen 310 an dem Gelenk 220, das durch den Niet 224 an dem proximalen Gelenkarmende 118 gebildet ist, verschwenkbar gelagert.

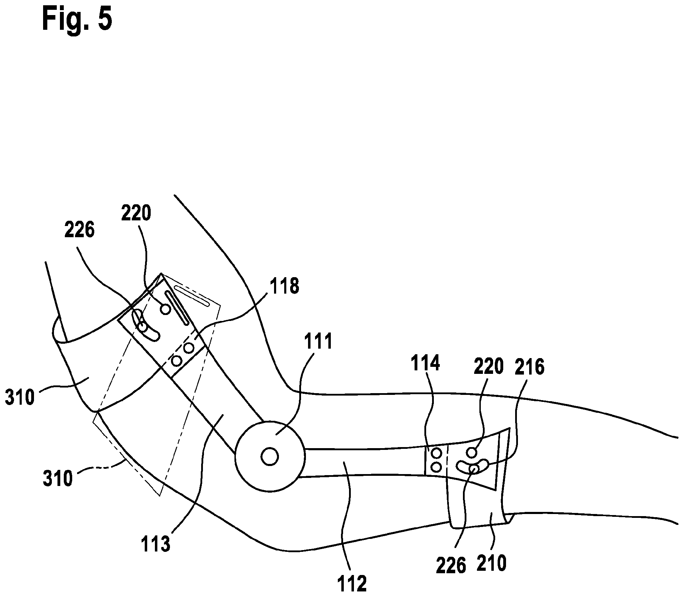

Figur 5 zeigt eine Ausführung als Handgelenkorthese. Die Bezugszeichen aus den Figuren 1 bis 4 gelten für entsprechende Strukturen. In dem Hartrahmen nach Figur 5 ist der proximale Brückenbogen 310 über das Gelenk 220 an dem proximalen Gelenkarmende 118 verschwenkbar gelagert, und der distale Brückenbogen 210 ist über das Gelenk 220 an dem distalen Gelenkarmende 114 verschwenkbar gelagert.

## Patentansprüche

1. Hartrahmen (100) für eine gelenküberbrückende Extremitätengelenkorthese, enthaltend
zwei sich gegenüber liegende längsverlaufende Gelenkschienen (110,120), die über deren proximale Gelenkarme (113, 123) über einen querenden proximalen Brückenbogen (310) miteinander verbunden sind und dort eine proximale Extremitätenfassung (300) bilden und weiter über deren distale Gelenkarme (112,122) über einen querenden distalen Brückenbogen (210) miteinander verbunden sind und dort eine distale Extremitätenfassung (200) bilden,
wobei mindestens einer der Brückenbögen (210) starr ist und jeweils an Gelenkarmenden (114,124) der verbundenen Gelenkarme (112,122) in Gelenken (220) in mindestens einer Verschwenkachse (222) schwenkbar gelagert ist und sich diese Verschwenkachse (222) in Richtung der Verbindung der beiden über diesen Brückenbogen (210) verbundenen Gelenkarme (112,122) erstreckt, **dadurch gekennzeichnet, dass** das Gelenk (220) für die Verschwenkung in der mindestens einen Verschwenkachse (222) eine Verschwenkungslimitierung (223) aufweist und die Verschwenkungslimitierung (223) des Gelenks (220) jeweils durch ein Langloch (216) in den Endabschnitten (212) des starren Brückenbogens (210) und einen an dem jeweiligen Gelenkarmende (114,124) feststehenden Gleitzapfen (226), der in dem jeweiligen Langloch (216) gleitet, gebildet ist und
wobei mindestens ein Gelenkarmende (114,124) als Federblatt ausgebildet ist, das eine federelastische Verkippung dieses Gelenkarmendes (114,124) aus der primären Schwenkebene der Gelenkschiene (110,120) erlaubt.

2. Hartrahmen (100) nach Anspruch 1, wobei die Gelenke (220) für die Verschwenkung in der mindestens einen Verschwenkachse (222) jeweils zwischen Endabschnitten (212) des starren Brückenbogens (210) und den damit überlappenden Gelenkarmenden (114,124) gebildet sind, wobei die Endabschnitte (212) mit den Gelenkarmenden (114,124) jeweils mit einem Achsbolzen (224) drehbar miteinander verkoppelt sind.

3. Hartrahmen (100) nach einem der vorstehenden Ansprüche, wobei das Gelenk (220) eine mechanische Hemmung aufweist, die der Verschwenkung einen Widerstand entgegensetzt.

4. Hartrahmen (100) nach Anspruch 3, wobei die Hemmung durch Rastervorsprünge, die zwischen Endabschnitten (212) des starren Brückenbogens (210) und den jeweils damit überlappend verbundenen Gelenkarmenden (114,124) ausgebildet sind, bewirkt ist.

5. Hartrahmen (100) nach Anspruch 4, wobei die Hemmung durch mechanische Reibung zwischen Endabschnitten (212) des starren Brückenbogens (210) und der jeweils damit überlappend verbundenen Gelenkarmenden (114,124) bewirkt ist.

6. Hartrahmen (100) nach Anspruch 5, wobei die mechanische Reibung durch einen an den Endabschnitten (212) und/oder an Gelenkarmenden (114,124) ausgebildeten Reibbelag (218) modifiziert ist.

7. Hartrahmen (100) nach einem der vorstehenden Ansprüche, wobei Endabschnitte (212) des starren Brückenbogens (210) und die jeweils damit verbundenen Gelenkarmenden (114,124) über einen Elastomerblock, der als Torsionsgelenk wirkt, mit einander elastisch verkoppelt sind.

8. Hartrahmen (100) nach einem der vorstehenden Ansprüche, wobei in einer oder beiden Gelenkschienen (110,120) zusätzlich mindestens ein fixierbares Kippgelenk (115) ausgebildet ist, zur Verkippung mindestens eines der Gelenkarme (112,113,122,123) aus der primären Schwenkebene der jeweiligen Gelenkschiene (110,120).

9. Gelenkorthese, enthaltend den Hartrahmen (100) nach einem der vorstehenden Ansprüche und weiter enthaltend Gurtbänder (350, 250) zur Fixierung der zu stützenden Extremität an den jeweiligen proximalen und distalen Fassungen (300,200) des Hartrahmens (100).

10. Gelenkorthese nach Anspruch 9, die eine Kniegelenkorthese ist.

## Claims

1. A hard frame (100) for a joint bridging limb joint orthosis including
two oppositely disposed longitudinally extending joint bars (110, 120) which are interconnected via their proximal joint arms (113, 123) via a transverse proximal bridge arch (310) and form there a proximal limb socket (300) and are further interconnected via their distal joint arms (112, 122) via a transverse distal bridge arch (210) and form there a distal limb socket (200),
wherein at least one of the bridge arches (210) is rigid and is pivotally mounted at respective joint arm ends (114, 124) of the connected joint arms (112, 122) in joints (220) in at least one pivot axis (222) and this pivot axis (222) extends in the direction of the connection of the two joint arms (112, 122) connected via this bridge arch (210), **characterised in that in that** the joint (220) has a pivoting limitation (223) for the pivoting in the at least one pivot axis (222), and the pivoting limitation (223) of the joint (220) is formed in each case by an elongated hole (216) in the end sections (212) of the rigid bridge arch (210) and a sliding pin (226) which is fixed on the respective joint arm end (114, 124) and slides in the respective elongated hole (216), and
wherein at least one joint arm end (114, 124) is formed as a spring leaf which allows a spring-elastic tilting of this joint arm end (114, 124) from the primary pivoting plane of the joint bar (110, 120).

2. Hard frame (100) according to claim 1, wherein the joints (220) for pivoting in the at least one pivot axis (222) are each formed between end sections (212) of the rigid bridge arch (210) and the joint arm ends (114,124) overlapping therewith, wherein the end sections (212) with the joint arm ends (114,124) are each rotatably coupled to each other with an axle bolt (224).

3. Hard frame (100) according to any one of the preceding claims, wherein the joint (220) has a mechanical restraint which provides a resistance to pivoting.

4. Hard frame (100) according to claim 3, wherein the restraint is provided by grid projections formed between end sections (212) of the rigid bridge arch (210) and the respective joint arm ends (114,124) overlappingly connected thereto.

5. Hard frame (100) according to claim 4, wherein the restraint is provided by mechanical friction between end sections (212) of the rigid bridge arch (210) and the respective joint arm ends (114,124) overlappingly connected thereto.

6. Hard frame (100) according to claim 5, wherein the mechanical friction is modified by a friction lining (218) formed on the end sections (212) and/or on joint arm ends (114,124).

7. Hard frame (100) according to any one of the preceding claims, wherein end sections (212) of the rigid bridge arch (210) and the respective joint arm ends (114,124) connected thereto are elastically coupled to each other via an elastomeric block acting as a torsion joint.

8. Hard frame (100) according to one of the preceding claims, wherein at least one fixable tilting joint (115) is additionally formed in one or both joint bars (110, 120) for tilting at least one of the joint arms (112, 113, 122, 123) out of the primary pivoting plane of the respective joint bar (110, 120).

9. Joint orthosis comprising the hard frame (100) according to any one of the preceding claims and further comprising webbing straps (350, 250) for fixing the limb to be supported to the respective proximal and distal sockets (300, 200) of the hard frame (100).

10. A joint orthosis according to claim 9, which is a knee joint orthosis.

## Revendications

1. Un cadre dur (100) pour une orthèse de pontage articulaire d'un membre, contenant
deux rails d'articulé (110, 120) disposées de façon opposée et s'étendant longitudinalement, qui sont interconnectées par leurs bras d'articulé proximaux (113, 123) par l'intermédiaire d'un arc de pont proximal transversal (310) et y forment une douille de membre proximale (300) et sont en outre interconnectées par leurs bras d'articulé distaux (112, 122) par l'intermédiaire d'un arc de pont distal transversal (210) et y forment une douille de membre distale (200),
dans lequel au moins l'un des arcs de pont (210) est rigide et est monté de manière pivotante aux extrémités de bras d'articulés respectives (114, 124) des bras articulés (112, 122) reliés dans des articulations (220) dans au moins un axe de pivotement (222) et cet axe de pivotement (222) s'étend dans la direction de la liaison des deux bras articulés (112, 122) reliés par cet arc de pont (210), **caractérisé en ce que en ce que** l'articulation (220) présente une limitation de pivotement (223) pour le pivotement dans l'au moins un axe de pivotement (222), et la limitation de pivotement (223) de l'articulation (220) est formée à chaque fois par un trou oblong (216) dans les sections d'extrémité (212) de l'arc de pont rigide (210) et par une broche de glissement (226) qui est fixée sur l'extrémité de bras d'articulé respective (114, 124) et glisse dans le trou oblong respectif (216), et
dans lequel au moins une extrémité de bras articulé (114, 124) est formée comme une lame de ressort qui permet un basculement élastique de cette extrémité de bras articulé (114, 124) à partir du plan de pivotement primaire du rail articulé (110, 120).

2. Cadre dur (100) selon la revendication 1, dans lequel les articulations (220) pour le pivotement dans le au moins un axe de pivotement (222) sont chacune formées entre des sections d'extrémité (212) de l'arc de pont rigide (210) et les extrémités de bras d'articulés (114, 124) se chevauchant avec celui-ci, dans lequel les sections d'extrémité (212) avec les extrémités de bras d'articulés (114, 124) sont chacune couplées de manière rotative les unes aux autres avec un axe (224).

3. Cadre dur (100) selon l'une quelconque des revendications précédentes, dans lequel l'articulation (220) comprend une retenue mécanique qui oppose une résistance au pivotement.

4. Le cadre dur (100) de la revendication 3, dans lequel la retenue est assurée par des saillies de verrouillage formées entre des sections d'extrémité (212) de l'arc de pont rigide (210) et les extrémités de bras articulés respectives (114, 124) reliées à celles-ci en se chevauchante.

5. Cadre dur (100) selon la revendication 4, dans lequel la retenue est assurée par un frottement mécanique entre les sections d'extrémité (212) de l'arc de pont rigide (210) et les extrémités de bras articulés respectives (114, 124) reliées à celles-ci en se chevauchant.

6. Cadre dur (100) selon la revendication 5, dans lequel le frottement mécanique est modifiée par une garniture de frottement (218) formée sur les sections d'extrémité (212) et/ou sur les extrémités des bras articulé (114, 124).

7. Cadre dur (100) selon l'une quelconque des revendications précédentes, dans lequel les parties d'extrémité (212) de l'arc de pont rigide (210) et les extrémités de bras articulés respectives (114, 124) qui y sont reliées sont couplées élastiquement les unes aux autres par l'intermédiaire d'un bloc élastomère agissant comme une articulation de torsion.

8. Cadre dur (100) selon l'une des revendications précédentes, dans lequel au moins une articulation basculante (115) fixable est en outre formée dans l'un ou les deux rails d'articulation (110, 120) pour faire basculer au moins l'un des bras d' articulés (112, 113, 122, 123) hors du plan de pivotement primaire du rail d'articulation respectif (110, 120).

9. Orthèse articulaire comprenant le cadre dur (100) selon l'une quelconque des revendications précédentes et contentant en outre des sangles (350, 250) pour fixer le membre à supporter aux douilles proximale et distale respectives (300, 200) du cadre dur (100).

10. Orthèse articulaire selon la revendication 9, qui est une orthèse articulaire du genou.
